# EUROPÄISCHE PATENTSCHRIFT

(11) **EP 2 344 197 B1**
(45) Veröffentlichungstag und Bekanntmachung des Hinweises auf die Patenterteilung: **05.04.2017**
(21) Anmeldenummer: 09736157.0
(22) Anmeldetag: 21.09.2009
(51) Int. Cl.: A61K 47/26, A61K 47/38, A61K 31/522, A61K 9/20, A61K 31/00

(54) **TABLETTIERHILFSSTOFF AUF LAKTOSE- UND CELLULOSEBASIS**
LACTOSE AND CELLULOSE-BASED TABLETING AID
AUXILIAIRE DE PASTILLAGE À BASE DE LACTOSE ET DE CELLULOSE

(30) Priorität: 19.09.2008 DE 102008047910
(43) Veröffentlichungstag der Anmeldung: 20.07.2011
(73) Patentinhaber: Molkerei Meggle Wasserburg GmbH & Co. Kg, 83512 Wasserburg (DE)
(72) Erfinder: SCHWARZ, Eugen, 83512 Wasserburg (DE); WARNKE, Gernot, 83123 Amerang (DE); FICHTNER, Vera, 83512 Reitmehring (DE)
(74) Vertreter: Weickmann & Weickmann PartmbB
(86) Internationale Anmeldenummer: PCT/EP2009/062203
(87) Internationale Veröffentlichungsnummer: WO 2010/031866

(56) Entgegenhaltungen:
- EP-A2- 0 948 321
- WO-A2-2007/138606
- US-A- 4 693 750
- US-A1- 2007 014 853
- TAKEUCHI H ET AL: "Temperature-induced crystallization and compactibility of spray dried composite particles composed of amorphous lactose and various types of water-soluble polymer" CHEMICAL AND PHARMACEUTICAL BULLETIN, PHARMACEUTICAL SOCIETY OF JAPAN, TOKYO, JP, Bd. 48, Nr. 4, 1. April 2000 (2000-04-01), Seiten 585-588, XP009127441 ISSN: 0009-2363
- REMON J P ET AL: "EFFECT OF RAW MATERIALS AND PROCESSING ON THE QUALITY OF GRANULES PREPARED FROM MICROCYSTALLINE CELLULOSE-LACTOSE MIXTURES" DRUG DEVELOPMENT AND INDUSTRIAL PHARMACY, NEW YORK, NY, US, Bd. 13, Nr. 1, 1. Januar 1987 (1987-01-01) , Seiten 1-14, XP002052285 ISSN: 0363-9045
- CHAMBIN O ET AL: "Effects of different cellulose derivatives on drug release mechanism studied at a preformulation stage", JOURNAL OF CONTROLLED RELEASE, ELSEVIER, AMSTERDAM, NL, vol. 95, no. 1, 20 February 2004 (2004-02-20), pages 101-108, XP004487819, ISSN: 0168-3659, DOI: 10.1016/J.JCONREL.2003.11.009

## Beschreibung

Die vorliegende Erfindung betrifft ein Verfahren zur Herstellung eines Granulats auf Basis von Laktose und Cellulose(derivaten), ein durch das Verfahren erhältliches Granulat und dessen Verwendung als Tablettierhilfsstoff.

Tabletten werden aus technologischer Sicht als einzeln dosierte feste Arzneiformen definiert, die durch Komprimieren von Pulvern oder Granulaten in verschiedensten Formen hergestellt werden. Die Zusammensetzung von Tabletten kann äußerst vielfältig sein und muss individuell für jeden Wirkstoff, für jeden Verwendungszweck und für jede Herstellungstechnologie entwickelt werden.

Typische Tablettenformulierungen enthalten neben der pharmazeutisch aktiven Komponente sogenannte Tablettierhilfsstoffe, wie z.B. Füllstoffe (Milchzucker, Cellulosepulver, Kalziumdiphosphat, mikrokristalline Cellulose, Zuckeralkohole, z.B. Mannit, Sorbit und Stärke), Sprengmittel (Stärke(derivate), Croscarmellose, quervernetztes PVP, Carboxymethylcellulose), Schmiermittel (Stearinsäure, Magnesiumstearat), Gleitmittel (Siliciumdioxid (Aerosil)) oder Mischungen davon. Tablettierhilfsstoffe sind Additive, die eine praktikable Herstellung von Tabletten erst möglich machen und haben einen wesentlichen Einfluss auf die Verarbeitbarkeit der Tablettenformulierung und auf die Eigenschaften der fertigen Tablette. Die Tablettierhilfsstoffe werden je nach Darreichungsform und eingesetzter aktiven Komponente ausgewählt.

Üblicherweise werden die pharmazeutisch aktiven Komponenten zusammen mit den jeweiligen Tablettierhilfsstoffen unter Zuhilfenahme eines Lösungsmittels zu einem Granulat verarbeitet, das in einem weiteren Schritt zu einer Tablette verpresst wird.

Der einfachste und ökonomischste Weg zur Tablettenherstellung ist jedoch die Direkttablettierung, d.h. eine Tablettierung ohne vorhergehende Granulation von aktiver/aktiven Komponente(n) und Tablettierhilfsstoffen. Tablettenformulierungen, die für die Direkttablettierung geeignet sind, müssen eine ausreichende plastische Verformbarkeit und gute Fließeigenschaften haben und dürfen keine Entmischungstendenzen zeigen. Diese drei Voraussetzungen zu beherrschen, ist außerordentlich schwierig, weswegen die Direkttablettierung bisher nur selten durchgeführt werden kann (K. Bauer, Pharmazeutische Technologie, 1993, Georg Thieme Verlag, Stuttgart).

Bei direkt verpressbaren Tablettenformulierungen sollte die Korngröße der pharmazeutisch aktiven Komponente und der Direkt(Tablettierhilfsstoff) zwischen 10 und 1000 µm liegen, um eine Entmischung der Bestandteile in der Tablettenformulierung zu minimieren. Unterschiedliche Korngrößenverteilung von pharmazeutisch aktiven Komponenten, Direkttablettierhilfsstoffen und gegebenenfalls weiteren Hilfsstoffen sind insbesondere dann kritisch, wenn die Tablettenformulierung aus mindestens drei Komponenten besteht.

Neben der Kosteneffektivität ist aber ein weiterer Vorteil der Direkttablettierung, dass keine Granulation der pharmazeutisch aktiven Komponente nötig ist und deswegen auch lösungsmittelempfindliche Komponenten ohne Weiteres verarbeitet werden können.

Es besteht daher ein großer Bedarf an Tablettierhilfsstoffen, die mit der pharmazeutisch aktiven Komponente und gegebenenfalls weiteren Tablettierhilfsstoffen einfach vermischt und anschließend direkt verpresst werden können (Direkttablettierhilfsstoff).

Das oben beschriebene Eigenschaftsprofil von direkt verpressbaren Tablettenformulierungen wird durch bloßes Mischen der kommerziell erhältlichen Einzelbestandteile einer Tablettenformulierung (physikalische Mischung) in den meisten Fällen nicht erreicht. Häufig werden daher Mischgranulate aus verschiedenen Tablettierhilfsstoffen eingesetzt.

Derartige Mischgranulate eignen sich insbesondere zur Verwendung als Direkttablettierhilfsstoff, bieten aber auch Vorteile als Tablettierhilfsstoff bei der konventionellen Herstellung von Tabletten.

US 6,770,368 beschreibt ein Granulat aus Stärke und Laktose als Hilfsmittel für die Direkttablettierung. Hierzu wird eine Lösung oder Suspension aus den beiden Komponenten in einem Sprühtrocknungsverfahren getrocknet.

US 4,693,750 beschreibt ein Hilfsmittel für die Direkttablettierung, das im Wesentlichen aus Laktose und Cellulose besteht. Hierzu wird Cellulosepulver und Laktose in heißem Wasser vermischt und anschließend sprühgetrocknet. Das erhaltene Pulver zeichnet sich durch seine Fließeigenschaften und - in gepresster Form - durch seine Tablettenhärte aus.

EP 0 948 321 offenbart die Herstellung einer Laktose/Ethylcellulosezubereitung, bei der die beiden Komponenten mit Hilfe eines Rührers in Wasser dispergiert werden und anschließend in einem Labor-Sprühturm versprüht werden. Ein gut fließfähiges Sprüh-Agglomerat wird erhalten und u. a. als Direkttablettierhilfsstoff eingesetzt.

Laktose (Milchzucker) wird heute in großem Umfang als Tablettierhilfsstoff u. a. in der Pharmazie, im Lebensmittelbereich, aber auch in der technischen Industrie eingesetzt. Laktose gehört zur Gruppe der Disaccharide und besteht aus den beiden Molekülen β-D-Galaktose und α/β-D-Glucose, die über eine β-1,4-glykosidische Bindung miteinander verbunden sind.

Ein Vorteil von Laktose als Tablettierhilfsstoff ist deren geringe Hygroskopizität, der günstige Preis, die gute Wasserlöslichkeit und die Reaktionsträgheit gegenüber den meisten pharmazeutisch aktiven Komponenten.

Laktose ist auf den Markt in zwei Modifikationen, nämlich als wasserfreie Laktose und als Laktose-Monohydrat erhältlich. Laktose-Monohydrat wird bevorzugt, da es im Vergleich zu wasserfreier Laktose weniger hygroskopisch ist und somit in Zusammensetzungen, die wasserempfindliche pharmazeutisch aktive Komponenten enthalten, besser geeignet ist.

Cellulose ist ein Polysaccharid, das aus einer Vielzahl von β-D-Glucosemolekülen, die über eine 1,4-β-glykosidische Bindung verknüpft sind, besteht. Die in dem Polysaccharid enthaltenen Hydroxylgruppen können auf vielfältige Weise chemisch umgesetzt werden. So können die Hydroxylgruppen der Cellulose unabhängig voneinander zumindest teilweise unter bestimmten Reaktionsbedingungen alkyliert, hydroxyalkyliert, sulfoniert, nitriert, carboxyalkyliert oder/und xanthogeniert werden.

Die so erhaltenen modifizierten Cellulosen stellen Cellulosederivate dar, deren Eigenschaftsprofile, z.B. hinsichtlich der Wasserlöslichkeit und Wirkstoffkompatibilität für die jeweilige Anwendung maßgeschneidert werden können.

Cellulose und Cellulosederivate, insbesondere Hypromellose (Hydroxypropylmethylcellulose (HPMC)), Hypromellosephtalat, Hydroxypropylcellulose (HPC), Hydroxyethylcellulose (HEC), Carboxymethylcellulose (CMC), Carboxyethylcellulose (CEC), Ethylcellulose (EC) sowie Salze davon eignen sich als Hilfsmittel bei der Tablettenformulierung.

Zur Herstellung von Retard-Tablettenkernen, ist es wünschenswert den Gehalt an Cellulosederivaten auf mindestens 15 %, bevorzugt mindestens 20 % zu erhöhen. In dieser Konzentration ist jedoch die Fließeigenschaft der Formulierung oftmals sehr eingeschränkt und eine Verarbeitung der Formulierung zu Tabletten, insbesondere eine Direktverpressung, erschwert oder gar nicht möglich.

Im Hinblick auf den Stand der Technik ist es daher wünschenswert Tablettierhilfsstoffe und insbesondere Direkttablettierhilfsstoffe bereitzustellen, durch die das Eigenschaftsprofil von Tablettenformulierungen hinsichtlich Fließverhalten und/oder Verpressbarkeit und das Eigenschaftsprofil der daraus hergestellten Tabletten hinsichtlich Tablettenhärte, Abriebbeständigkeit, Freisetzungsprofil und/oder Presskraft-Härte-Profil weiterhin verbessert wird.

Die vorliegende Erfindung stellt dementsprechend ein Verfahren zur Herstellung eines Granulats bereit, umfassend die Schritte
i) Suspendieren oder/und zumindest teilweises Lösen von Laktose in einer Flüssigkeit, ausgewählt aus Wasser oder einem organischen Lösungsmittel, und
ii) Zerstäuben der in i) erhaltenen Lösung oder Suspension in eine Umgebung mit einer Temperatur von 30-250 °C auf Cellulosederivat-Partikel und gegebenenfalls Laktose-Partikel, wobei die Flüssigkeit zumindest teilweise entfernt wird.

Es wurde gefunden, dass die Fließeigenschaften und die Partikelgrößen des erfindungsgemäßen Granulats im Schritt ii) leicht so eingestellt werden können, dass sie eine einfache Direkttablettierung, die mit einer physikalischen Mischung der entsprechenden Komponenten nicht möglich ist, zulassen. Überraschenderweise können ferner durch Verwendung des erfindungsgemäßen Granulats aus Laktose und Cellulosederivat bei der Direkttablettierung Tabletten erhalten werden, deren Abriebfestigkeit und Tablettenhärte bei gleichem Verpressungsdruck signifikant gegenüber einer Tablette, bei der eine physikalische Mischung der Granulatbestandteile verwendet wird, erhöht sind.

Für das erfindungsgemäße Verfahren kann Laktose wasserfrei oder als Laktose-Monohydrat verwendet werden. Bevorzugt wird Laktose-Monohydrat aufgrund der bereits erwähnten geringeren Hygroskopizität im Vergleich zu wasserfreier Laktose verwendet.

Die in Schritt ii) und gegebenenfalls in Schritt i) verwendete Cellulosederivate können unabhängig voneinander ausgewählt werden und gleich oder unterschiedlich sein.
Cellulose wird bevorzugt aus natürlichen Quellen gewonnen und ist gegebenenfalls in weiteren Schritten aufgereinigt worden.
Cellulosederivate sind chemisch modifizierte Cellulosen, bei denen die Hydroxylgruppen unabhängig voneinander zumindest teilweise alkyliert, hydroxyalkyliert, sulfoniert, nitriert, carboxyalkyliert oder/und xanthogeniert sind.

Insbesondere werden in dem erfindungsgemäßen Verfahren natürliche Cellulose oder/und Cellulosederivate oder Mischungen davon eingesetzt, bei denen die Hydroxylgruppen der Cellulose unabhängig voneinander zumindest teilweise alkyliert, hydroxyalkyliert, sulfoniert, carboxyalkyliert oder/und xanthogeniert sind. Besonders bevorzugt werden in dem erfindungsgemäßen Verfahren Cellulosederivate eingesetzt, bei denen die Hydroxylgruppen der Cellulose unabhängig voneinander zumindest teilweise methyliert, ethyliert, hydroxypropyliert, hydroxypropylmethyliert, hydroxyethyliert, carboxymethyliert oder/und carboxyethyliert sind.

Aufgrund ihrer guten Verpressbarkeit werden bevorzugt Celluloseether als Cellulosederivate verwendet. Als Beispiele seien Hypromellose (Hydroxypropylmethylcellulose (HPMC), Hypromellosephthalat, Hydroxypropylcellulose (HPC), Hydroxyethylcellulose (HEC), Carboxymethylcellulose (CMC), Carboxyethylcellulose (CEC), Ethylcellulose (EC) sowie Salze davon (Natrium- oder/und Calciumsalze) genannt.

Besonders bevorzugt wird Hypromellose (HPMC), Hydroxypropylcellulose (HPC) und Hydroxyethylcellulose (HEC), und insbesondere Hypromellose (HPMC) eingesetzt.

Das Molekulargewicht der Cellulosederivate kann in weiten Bereichen variieren und liegt bevorzugt zwischen 1 x 10³ und 2 x10⁶ g/mol und stärker bevorzugt zwischen 5 x 10⁵ und 1,5 x 10⁶g/mol (Mₙ).

Laktose wird in einer Flüssigkeit suspendiert oder/und zumindest teilweise gelöst. Als Flüssigkeit kann Wasser oder ein organisches Lösungsmittel verwendet werden, das unter bestimmten Druck- und Temperaturbedingungen in einem flüssigen Aggregatzustand vorliegt und inert gegenüber Laktose ist.

Als Flüssigkeit können Wasser oder organische Lösungsmittel verwendet werden. Geeignete organische Lösungsmittel sind beispielsweise Methanol, Ethanol oder Aceton. In einer anderen Ausführungsform können auch Flüssigkeitsgemische eingesetzt werden.

Im Schritt i) werden Wasser, Ethanol sowie
Mischungen davon verwendet. Eine besonders bevorzugte Flüssigkeit ist Wasser.

Zur Herstellung der Lösung oder/und Suspension wird Laktose z.B. unter mechanischem Rühren, in eine Flüssigkeit eingearbeitet. Zum Einarbeiten werden übliche Rührvorrichtungen verwendet.

Um das Lösen der Ausgangsmaterialien zu beschleunigen, kann die Flüssigkeit auf 30°C bis 90°C, bevorzugt auf 40°C bis 70°C während des Einarbeitungsschritts erwärmt werden.

Im Schritt i) ist es bevorzugt, dass mindestens 5 Gew.-%, bevorzugt mindestens 20 Gew.-%, stärker bevorzugt mindestens 80 Gew.-%, am stärksten bevorzugt 100 Gew.-% bezogen auf den Gesamtgehalt an Laktose in gelöster Form in der Flüssigkeit vorliegen.

Die durchschnittliche Partikelgröße einer in i) erhaltenen Suspension sollte im Bereich zwischen 0,1 µm bis etwa 1000 µm, bevorzugt zwischen 1 µm bis 500 µm, besonders bevorzugt zwischen 2 µm bis 200 µm liegen.

Die im Schritt i) erhaltene Lösung oder/und Suspension, die eine Temperatur von 20 bis 90°C, bevorzugt von 20 bis 70°C, stärker bevorzugt von 40 bis 70 °C aufweisen kann, wird anschließend im Schritt ii), z.B. über eine Düse, zu Tröpfchen mit einem durchschnittlichen Durchmesser von 15 µm bis 1250 µm, bevorzugt von 20 µm bis 1000 µm, besonders bevorzugt von 40 µm bis 750 µm in eine Umgebung mit einer Temperatur von 30 bis 250°C, bevorzugt von etwa 40 bis 170°C, zerstäubt.

Der Druck in der Umgebung, in die die Tröpfchen eingebracht werden liegt im Bereich von etwa 0 bis 1,0 bar, bevorzugt von 0,003 bis 0,7 bar, besonders bevorzugt von 0,005 bis 0,5 bar.

Geeignete Zerstäuber-Düsen sind beispielsweise Ein-Stoff-, Zwei-Stoff oder Mehr-Stoff-Druckdüsen, wie z.B. Turbulenz-, Flachstrahl-, Prall- oder Hohlkegeldruckdüsen, pneumatische Düsen, aber auch Ultraschalldüsen. In einer bevorzugten Ausführungsform werden Einstoffdüsen bei einem Düsendruck von 20 bis 250 bar, bevorzugt von 30 bis 200 bar, und Zwei- bzw. Mehrstoffdüsen bei einem Düsendruck von 0,1 bis 10 bar, bevorzugt von 0,3 bis 5 bar betrieben.

Das Zerstäuben einer Flüssigkeit oder/und Suspension in eine Umgebung mit erhöhter Temperatur und gegebenenfalls reduziertem Druck, führt dazu, dass die Flüssigkeit aus den Tröpfchen zumindest teilweise entfernt wird. Dieses Verfahren ist in der Technik als Sprühtrocknung bekannt.

Die im Schritt i) erhaltene Lösung oder/und Suspension wird auf Cellulosederivat-Partikel und gegebenenfalls Laktose-Partikel zerstäubt. Die Cellulosederivat-und Laktose-Partikel haben einen durchschnittlichen Durchmesser von etwa 1 µm bis etwa 500 µm, bevorzugt von 2 µm bis 300 µm, besonders bevorzugt von 5 µm bis 200 µm.

Ein bevorzugtes Gewichts-Verhältnis der Cellulosederivatzu Laktose-Partikel in Schritt ii) liegt im Bereich von 100/0 bis 5/95, besonders bevorzugt von etwa 100/0 bis etwa 50/50. In einer bevorzugten Ausführungsform wird die in i) erhaltene Suspension oder/und Lösung nur auf Cellulosederivat-Partikel zerstäubt (Cellulosederivat-/Laktose-Partikel ist 100/0).

In einer Ausführungsform können sich die Cellulosederivat-Partikel und gegebenenfalls Laktose-Partikel in einem geeigneten Mischer befinden, während die im Schritt i) erhaltene Lösung oder/und Suspension darauf zerstäubt wird. Unter den oben genannten Bedingungen der Feuchtgranulation wird die Flüssigkeit aus den Tröpfchen zumindest teilweise durch geeignete Trocknungsverfahren entfernt.

In einer anderen Ausführungsform wird die in Schritt i) erhaltene Suspension oder/und Lösung auf die Cellulosederivat-und gegebenenfalls Laktose-Partikel zerstäubt, während die Gesamtheit der Cellulosederivat-und
gegebenenfalls Laktose-Partikel in einem Fließbett bzw. einer Wirbelschicht vorliegen.

Die Wirbelschicht ist eine Schüttung aus Cellulosederivat-und
gegebenenfalls Laktose-Partikeln, die durch eine gerichtete Strömung eines Gases, in einen fluidisierten Zustand versetzt wird.

Beim Aufsprühen der in Schritt i) enthaltenen Suspension oder/und Lösung auf eine Wirbelschicht (Wirbelschicht-Granulationsverfahren) liegen die einzelnen Cellulosederivat- und gegebenenfalls Laktose-Partikel im Wesentlichen getrennt voneinander vor, sodass eine homogene und vollständige Verteilung der im Schritt i) erhaltenen Lösung oder/und Suspension auf der Oberfläche der fluidisierten Cellulosederivat-und gegebenenfalls Laktose-Partikel erreicht werden kann. Während des Wirbelschicht-Granulationsverfahrens wird die Flüssigkeit zumindest teilweise entfernt.

In einer anderen Ausführungsform wird die in Schritt i) erhaltene Suspension oder/und Lösung auf die Cellulosederivat-und gegebenenfalls Laktose-Partikel zerstäubt, die sich in einem Luftstrom befinden. Bei diesem Verfahren können die Feinanteile der Partikel zurückgeführt werden, um eine weitere Agglomeration der Partikel zu erzielen. Während dieses Verfahrens wird die Flüssigkeit zumindest teilweise entfernt.

In der vorliegenden Erfindung ist das Wirbelschicht-Granulationsverfahren bevorzugt.

In einer anderen Ausführungsform wird das Feuchtgranulationsverfahren bevorzugt.

In der Regel werden Druck- und Temperatur in der Umgebung so eingestellt, dass die Tröpfchen nicht schon vollständig getrocknet sind, bevor sie auf die Cellulose(derivat)- und gegebenenfalls Laktose-Partikel auftreffen. Dabei wird eine homogene Verteilung der im Schritt i) eingesetzten Lösung oder/und Suspension auf den Cellulosederivat-und gegebenenfalls
Laktose-Partikeln erreicht.

Nach dem Zerstäuben der in Schritt i) erhaltenen Lösung oder/und Suspension auf die Cellulosederivat- und gegebenenfalls Laktose-Partikel kann dem erhaltenen Produkt unter den Umgebungsbedingungen weiterhin Flüssigkeit entzogen werden bis der Gehalt an freier Flüssigkeit im Granulat bei < 8 Gew.-%, bevorzugt bei < 6 Gew.-%, insbesondere bevorzugt bei < 4 Gew.-% bezogen auf die Gesamtmasse des Granulats liegt.

Das erhaltene Granulat weist ein Verhältnis von Laktose zu Cellulosederivat zwischen etwa 95/5 bis 1/99, bevorzugt 90/10 bis 5/95 und stärker bevorzugt zwischen 60/40 bis 40/60 auf.

Die erhaltenen Granulatpartikel sind bevorzugt sphärisch oder sphäroid. Eine derartige Morphologie ist vorteilhaft für die Fließeigenschaften des Granulats. Die Granulatpartikel weisen eine d₅₀-Partikelgrößenverteilung von 25 bis 750 µm, bevorzugt von 30 bis 500 µm, stärker bevorzugt von 40 bis 350 µm auf. Dem Fachmann ist bewusst, dass die Partikelgröße des Granulats über die Verfahrensparameter (Umgebungsbedingungen, Sprührate, Partikelgröße in der Suspension, Partikelgröße der Cellulose(derivat)- bzw. Laktose-Partikel, etc.) in weiten Bereichen eingestellt werden kann.

Mit dem erfindungsgemäßen Verfahren kann ein Granulat aus Cellulosederivat und Laktose mit hohem Cellulosederivat-Anteil bereitgestellt werden.

Es wurde gefunden, dass die Fließfähigkeit des erfindungsgemäßen Granulats gegenüber der Fließfähigkeit der physikalischen Mischung deutlich verbessert ist.

Ein weiterer Gegenstand der Erfindung ist ein Granulat, das durch das oben beschriebene Verfahren erhältlich ist.

Darüber hinaus ist ein Gegenstand der vorliegenden Erfindung eine Zusammensetzung, die das erfindungsgemäße Granulat, mindestens eine pharmazeutisch aktive Komponente und gegebenenfalls weitere Hilfsstoffe umfasst.

Das Gewichtsverhältnis von Granulat zu pharmazeutisch aktiver Komponente kann in beliebigen Bereichen variieren und liegt bevorzugt zwischen 99,9 und 5, stärker bevorzugt zwischen 99 und 30 (Gewichtsverhältnis-Quotient). In einer anderen Ausführungsform liegt das Gewichtsverhältnis von Granulat zu pharmazeutisch aktiver Komponente zwischen 99,9 zu 0,1 und 20 zu 80.

Das Gewichtsverhältnis von Granulat zu Hilfsstoffen kann in beliebigen Bereichen variieren und liegt beispielsweise zwischen 100 und 0,5, bevorzugt zwischen 100 und 5 (Gewichtsverhältnis-Quotient). In einer anderen Ausführungsform liegt das Gewichtsverhältnis von Granulat zu Hilfsstoffen zwischen 100 zu 0 und 21 zu 79.

Geeignete Hilfsstoffe können z.B. Schmiermittel oder Gleitmittel, wie z. B. Stearinsäure, Magnesiumstearat oder Talkum, Füllmittel, wie z.B. Milchzucker, Cellulosepulver, mikrokristalline Cellulose, zusätzliche Cellulose(derivat)verbindungen, bevorzugt Hydroxypropylcellulose, oder Calciumdiphosphat, Fließregulierungsmittel, wie z. B. Siliciumdioxid (Aerosil^{®}), Antistatika, wie z. B. Aluminiumoxid, PEG, Lösungsvermittler, wie z. B. Saponine, und Feuchthaltemittel, wie z. B. Glycerin oder PEG, sein. Das erfindungsgemäße Granulat kann als Tablettierhilfsstoff verwendet werden. Dabei kann das erfindungsgemäße Granulat einerseits zusammen mit der pharmazeutisch aktiven Komponente und gegebenenfalls weiteren Tablettierhilfsstoffen granuliert werden, andererseits kann das erfindungsgemäße Granulat mit einem, die pharmazeutisch aktive Komponente enthaltenden, Granulat vermischt werden bevor die Formulierung verpresst wird.

Insbesondere kann das erfindungsgemäße Granulat als Direkttablettierhilfsstoff verwendet werden. Hierzu wird die pharmazeutisch aktive Komponente und gegebenenfalls weitere Tablettierhilfsstoffe einfach mit dem erfindungsgemäßen Granulat vermischt und direkt verpresst.

Es hat sich nämlich gezeigt, dass durch das erfindungsgemäße Verfahren ein Granulat erhalten wird, durch das der Gehalt an Cellulosederivaten in der Tablettenformulierung erhöht werden kann, ohne dass die Fließfähigkeit der Tablettenformulierung wesentlich beeinflusst wird.

Dies kann u.a. dadurch erklärt werden, dass die Oberfläche der Laktose- oder/und Cellulosepartikel durch das erfindungsgemäße Verfahren modifiziert wird, wodurch die Tendenz zur Agglomeration der Partikel stark verringert wird und entsprechend das Fließverhalten des Granulats bzw. der Tablettenformulierung verbessert wird.

Es hat sich gezeigt, dass der Einsatz des erfindungsgemäßen Granulats als (Direkt)Tablettierhilfsstoff in Standard-Tablettenformulierungen zu einer signifikanten Verbesserung der Tablettenhärte und der Abriebfestigkeit führt, im Vergleich zu Tabletten, bei deren Herstellung die Bestandteile des erfindungsgemäßen Granulats als einzelne Komponenten eingesetzt werden.

So ist die Tablettenhärte bei vergleichbarer Presskraft in der Regel in Granulat-haltigen Tabletten um mindestens 20 %, bevorzugt mindestens 50% gegenüber Tabletten, in denen die Granulatbestandteile als physikalische Mischung vorliegen, erhöht.

Der Abrieb bei Granulat-haltigen Tabletten ist in der Regel bei vergleichbarer Presskraft um mindestens 20 %, bevorzugt mindestens 50 % gegenüber Tabletten, in denen die Granulatbestandteile als physikalische Mischung vorliegen, verringert.

Durch Einsatz des erfindungsgemäßen Granulats als Tablettierhilfsstoff, insbesondere als Direkttablettierhilfsstoff kann das Presskraft-Härteprofil sowie das Presskraft-Abriebfestigkeitsprofil auf die jeweilige Anwendung eingestellt werden.

Es hat sich ferner gezeigt, dass bei Verwendung des erfindungsgemäßen Granulats als (Direkt)Tablettierhilfsstoff das Freisetzungsprofil einer pharmazeutisch aktiven Komponente gesteuert werden kann.

Für eine verzögerte Freisetzung der pharmazeutisch aktiven Komponente ist insbesondere der Anteil an Cellulosederivat in der Formulierung verantwortlich (s.o.). Aufgrund des in weiten Bereichen einstellbaren Gehalts an Cellulosederivat im Granulat und entsprechend in der Tablettenformulierung kann die Freisetzung einer pharmazeutisch aktiven Komponente eingestellt werden, ohne dass eine schlecht fließende Tablettenformulierung ein Direkttablettierverfahren unmöglich macht. Insbesondere eignet sich das erfindungsgemäße Granulat zur Verwendung in Retardformulierungen.

### Abbildungen

Abbildung 1 zeigt den Einfluss der Presskraft auf die Tablettenhärte in den Beispielen A und B.
Abbildung 2 zeigt den Einfluss der Presskraft auf den Abrieb in den Beispielen A und B.
Abbildung 3 zeigt den Einfluss der Presskraft auf die Tablettenhärte in den Beispielen C und D.
Abbildung 4 zeigt den Einfluss der Presskraft auf den Abrieb in den Beispielen C und D.
Abbildung 5 zeigt den Einfluss der Presskraft auf die Tablettenhärte in den Beispielen W1 - W3.
Abbildung 6 zeigt die Freisetzung von Theophyllin aus den Tabletten W1 - W3 in Abhängigkeit von der Zeit.
Abbildung 7a zeigt eine rasterelektronenmikroskopische (REM) Aufnahme der physikalischen Mischung B0.
Abbildung 7b zeigt eine REM-Aufnahme des Granulats B1.
Abbildung 8 zeigt die Partikelgrößenverteilung des Granulats B1.
Abbildung 9 zeigt das Fließverhalten des Granulats B1 (Ericksen Trichter Model 321, 6 mm Trichteröffnung).
Abbildung 10 zeigt die Freisetzung von Metformin HCl aus den Tabletten M1-M3 in Abhängigkeit von der Zeit in 0,1 M HCl.
Abbildung 11 zeigt die Freisetzung von Metformin HCl aus den Tabletten M1-M3 in Abhängigkeit von der Zeit in Acetat-Puffer (pH 4,5) USP.
Abbildung 12 zeigt die Freisetzung von Metformin HCl aus den Tabletten M1-M3 in Abhängigkeit von der Zeit in 0,05 M Phosphat-Puffer (pH 6,8) USP.

### Beispiele

### 1. Messmethoden

Die angegebenen Partikelgrößen wurden gemäß Ph. Eur. mit Rüttelsiebung bestimmt.

Der Carr-Index wird gemäß C = 100 [(V_{B} - V_{T})N_{B}], wobei V_{B} das Schüttvolumen und V_{T} das Stampfvolumen ist, errechnet und ist ein Maß für die Kompressibilität.

Sofern nicht anders angegeben wird die Fließfähigkeit der Formulierungen, die Abriebfestigkeit der Tabletten, die Tablettenhärte, das Schüttvolumen und die Stampfdichte wird anhand des Europäischen Arzneibuchs (PH. Eur) bestimmt.

Die Freisetzung wird mit Apparatur II (Erweka, Germany DT 808 LH) bestimmt. Die Tests finden in 1000ml 0,01 M HCl, 0,05 M Phosphat Puffer (pH 6,8) [hergestellt nach United States Pharmacopeial Convention (USP)] oder Acetat-Puffer (pH 4,5) [USP] bei Drehzahl 50 U/min statt. Die quantitative Messung des freigesetzten Wirkstoffs erfolgt durch UV-Spektroskopie.

Die Partikelgrößen(verteilungen) werden mit einem Sympatec Helios (H1511) im Messbereich R50,5/4,5 ...875 µm unter Verwendung eines Sympatec Rhodos Dispergiersystems gemessen. Der Dispersionsdruck beträgt 0.5 bar. Für die Zuführung wird eine Vibrationseinheit VIBRI (Trichterhöhe 2,5 mm, Leistung 60%) verwendet.

### 2. Herstellung des Granulats

### Beispiel A (Granulac 70: HPMC = 50:50)

62,5 g einer 40%-igen wässrigen Laktoselösung (25 g Laktose; Granulac 70, Meggle, Wasserburg) werden in einem Fließbettgranulator von Hüttlin Mycrolab auf 50 g HPMC-Partikel (Benecel K 4 M Pharm CR, Hercules) und 25 g Laktose (Granulac 70, Meggle, Wasserburg) zerstäubt. Die Granulierungsbedingungen sind in Tabelle 1 angegeben. Die Referenzprobe, bei der die entsprechenden Granulatbestandteile als physikalische Mischung vorliegen, wird mit der Probennr. A0 bezeichnet. Die Granulierbedingungen sind in Tabelle 1 zusammengefasst.

**Tabelle 1: Granulierungsparameter**

| **Probe** | **Einlassluftstrom [m³/h]** | **Einlasstemperatur [°C]** | **Umgebungstemperatur [°C]** | **Düsendruck [bar]** | **Umgebungsdruck [bar]** | **Sprührate [g/min]** |
|---|---|---|---|---|---|---|
| A1 | 17 | 80 | 48-52 | 0,4 | 0,11 | 1,8 |
| A2 | 17 | 70 | 42-46 | 0,4 | 0,11 | 2,2 |
| B1 | 16 | 80 | 52-54 | 0,41 | 0,2 | 2,4 |
| C1 | 17 | 80 | 52-54 | 0,4 | 0,1-0,2 | 2,8 |
| C2 | 17 | 80 | 50-53 | 0,4-0,5 | 0,11-0,15 | 2,2 |
| C3 | 15 | 68 | 41-43 | 0,4 | 0,11 | 3,6 |
| C4 | 17 | 46 | 33-35 | 0,4 | 0,11 | 1,9 |
| D1 | 13,5 | 80 | 52-55 | 0,45 | 0,2 | 2,2 |

### Beispiel B (Granulac 140: HPMC = 50:50)

62,5 g einer 40%-igen wässrigen Laktoselösung (25 g Laktose; Granulac 140, Meggle, Wasserburg) werden in einem Fließbettgranulator von Hüttlin Mycrolab auf 50 g HPMC-Partikel (Benecel K 4 M Pharm CR, Hercules) und 25 g Laktose (Granulac 140, Meggle, Wasserburg) zerstäubt. Die Granulierungsbedingungen sind in Tabelle 1 angegeben. Die Referenzprobe, bei der die entsprechenden Granulatbestandteile als physikalische Mischung vorliegen, wird mit der Probennr. B0 bezeichnet. Die Granulierbedingungen sind in Tabelle 1 zusammengefasst.

### Beispiel C (Granulac 70: HPMC = 40:60)

62,5 g einer 40%-igen wässrigen Laktoselösung (25 g Laktose; Granulac 70, Meggle, Wasserburg) werden in einem Fließbettgranulator von Hüttlin Mycrolab auf 60 g HPMC-Partikel (Benecel K 4 M Pharm CR Hercules) und 15 g Laktose (Granulac 70, Meggle, Wasserburg) zerstäubt. Die Granulierungsbedingungen sind in Tabelle 1 angegeben. Die Referenzprobe, bei der die entsprechenden Granulatbestandteile als physikalische Mischung vorliegen, wird mit der Probennr. C0 bezeichnet. Die Granulierbedingungen sind in Tabelle 1 zusammengefasst.

### Beispiel D (Granulac 140: HPMC = 40:60)

62,5 g einer 40%-igen wässrigen Laktoselösung (25 g Laktose; Granulac 140, Meggle, Wasserburg) werden in einem Fließbettgranulator von Hüttlin Mycrolab auf 60 g HPMC-Partikel (Benecel K 4 M Pharm CR Hercules) und 15 g Laktose (Granulac 140, Meggle, Wasserburg) zerstäubt. Die Granulierungsbedingungen sind in Tabelle 1 angegeben. Die Referenzprobe, bei der die entsprechenden Granulatbestandteile als physikalische Mischung vorliegen, wird mit der Probennr. D0 bezeichnet. Die Granulierbedingungen sind in Tabelle 1 zusammengefasst.

Die Eigenschaften der erhaltenen Granulate sowie der Ausgangsmaterialien sind in Tabelle 2 aufgeführt.

**Tabelle 2: Pulver- und Granulat-Eigenschaften**

| **Probe** | **Partikelgröße [µm]** | | | | | | | | | **Dichte [g/l]** | | **Carrindex** | **Fließfähigkeit [s/100g]** | | |
|---|---|---|---|---|---|---|---|---|---|---|---|---|---|---|---|
| | < 63 | 63-100 | 100-150 | 150-180 | 180-250 | 250-355 | 355-500 | 500-630 | > 630 | Schüttdichte | Stampfdichte | [%] | d= 10mm | d= 15mm | d= 25mm |
| Benecel K 4 M Pharm CR | 44,16 | 34 | 18,84 | 1,92 | 0,94 | 0,19 | 0,08 | 0,05 | 0,05 | 345 | 475 | 25,47 | ∞ | ∞ | ∞ |
| Granulac 70 | 8,98 | 26,91 | 30,06 | 15,13 | 17,8 | 1,4 | 0,23 | 0,04 | 0,04 | 699 | 877 | 20,3 | ∞ | ∞ | 2,3 |
| Granulac 140 | 11,24 | 26,64 | 32,94 | 22,51 | 6,33 | 0,47 | 0,27 | 0,08 | 0,05 | 613 | 862 | 28,89 | ∞ | ∞ | ∞ |
| A0 | 23,3 | 26,75 | 29,72 | 11,42 | 8,28 | 0,98 | 0,51 | 0,16 | 0,12 | 463 | 606 | 23,6 | ∞ | 7,53 | n/a |
| A1 | 12,14 | 26,65 | 35,12 | 15,06 | 10,46 | 1,14 | 0,17 | 0,12 | 0,2 | 467 | 575 | 18,78 | 21,47 | 6,83 | n/a |
| A2 | 11,63 | 24,07 | 32,81 | 15,24 | 13,37 | 2,05 | 0,56 | 0,3 | 0,88 | 459 | 568 | 19,19 | 22,2 | 6,93 | n/a |
| B0 | 32,69 | 36,06 | 24,57 | 4,28 | 1,92 | 0,46 | 0,26 | 0,21 | 0,12 | 467 | 641 | 27,15 | ∞ | ∞ | ∞ |
| B1 | 23,44 | 27,83 | 24,04 | 7,79 | 6,77 | 2,91 | 1,44 | 0,92 | 5,58 | 478 | 578 | 17,3 | n/a | n/a | n/a |
| C0 | 26,86 | 27,99 | 28,08 | 9,27 | 6,88 | 0,91 | 0,28 | 0,11 | 0,1 | 439 | 571 | 23,12 | ∞ | 8,83 | n/a |
| C1 | 10,5 | 21,23 | 32,18 | 15,7 | 17,17 | 3,7 | 0,38 | 0,04 | 0,1 | 397 | 469 | 15,35 | n/a | n/a | n/a |
| C2 | 10,41 | 21,94 | 32,44 | 16,3 | 16,48 | 2,64 | 0,44 | 0,2 | 0,25 | 413 | 478 | 13,6 | 24,3 | 7,53 | n/a |
| C3 | 13,36 | 21,97 | 27,62 | 14,57 | 17,67 | 4,91 | 0,63 | 0,05 | 0,09 | 422 | 510 | 17,25 | 24,37 | 7,5 | n/a |
| C4 | 8,7 | 19,43 | 31,18 | 19,5 | 19,01 | 1,91 | 0,15 | 0,1 | 0,6 | 394 | 467 | 15,63 | 23,47 | 8,07 | n/a |
| D0 | 36,69 | 34,86 | 22,68 | 4,11 | 1,82 | 0,46 | 0,11 | 0 | 0,1 | 435 | 602 | 27,74 | ∞ | ∞ | ∞ |
| D1 | 27,04 | 29,15 | 22,28 | 6,58 | 6,79 | 4,07 | 1,55 | 0,5 | 3,11 | 457 | 578 | 20,93 | n/a | n/a | n/a |

| | | | | | | | | | | | | | | | |
|---|---|---|---|---|---|---|---|---|---|---|---|---|---|---|---|
| n/a ... not applicable | | | | | | | | | | | | | | | |

### Beispiel E (Granulac 200:HPMC = 60:40)

90 l Wasser werden in einem Ansatzbehälter auf 80°C +/- 10°C erhitzt und anschließend werden 60 kg Laktose (z.B. Granulac 200) darin gelöst. In einem Granulator (z.B. Fielder Aeromatic) werden 100 kg Benecel (K 4 M Pharm CR, Hercules) und 90 kg Laktose (Granulac 200) für ca. 5 min durch Einblasen von Luft gemischt. Anschließend erfolgt das Aufsprühen der Laktoselösung mit durchschnittlich 90 l /h (Druck der Zerstäubungsluft 3 bar). bei einer Zulufttemperatur von 120+/-10°C. Nach Beendigung des Granulationsschrittes erfolgt die Trocknung des Granulats bei einer Zulufttemperatur von 130 +/- 10°C. Getrocknet wird bis die Ablufttemperatur mindestens 85°C erreicht.

### 3. Herstellung der Tabletten

### 3.1. Tabletten ohne Wirkstoff

### 3.1.1 Formulierung mit Granulat

Die erhaltenen Granulate (Beispiele A bis E) werden in einem Turbula-Mischer (Fa. Bachofen WAB T2F) 5 Minuten gemischt. Anschließend wird Magnesiumstearat im Gewichtsverhältnis 99,5 : 0,05 zugegeben und eine weitere Minute gemischt. Die erhaltene Mischung wird dann tablettiert.

### 3.1.2 Formulierung mit physikalischer Mischung

Die in Tabelle 3 aufgeführten Komponenten (außer Magnesiumstearat) werden im jeweiligen Gewichtsverhältnis miteinander in einem Turbula-Mischer (Firma Bachofen WAB T2F) 5 Minuten gemischt. Anschließend wird Magnesiumstearat zugegeben und wiederum eine Minute gemischt. Die erhaltene Formulierung wird anschließend tablettiert.

**Tabelle 3: Zusammensetzung der Tabletten ohne Wirkstoff (physikalische Mischung)**

| Substanz | Formulierung | | | |
|---|---|---|---|---|
| | A 0 | B 0 | C 0 | D 0 |
| Benecel K 4 M Pharm CR | 49,75% | 49,75% | 59,75% | 59,75% |
| Granulac 70 | 49,75% | | 39,75% | |
| Granulac 140 | | 49,75% | | 39,75% |
| Magnesiumstearat | 0,5% | 0,5% | 0,5% | 0,5% |

### 3.2 Tabletten mit Wirkstoff Theophyllin

Die in Tabelle 4 aufgeführten Komponenten (außer Magnesiumstearat) werden im jeweiligen Gewichtsverhältnis in einem Turbula-Mischer (Firma Bachofen WAB T2F) 5 Minuten gemischt. Anschließend wird Magnesiumstearat zugegeben und wiederum eine Minute gemischt. Die erhaltene Mischung wird anschließend tablettiert.

**Tabelle 4: Tablettenformulierung mit Wirkstoff Theophyllin**

| Substanz | | | |
|---|---|---|---|
| | W1* | W2* | W3* |
| Theophyllin | 24,5% | 24,5% | 24,5% |
| Benecel K 4 M Pharm CR | 30% | | |
| MCC | | | |
| Granulat E | | 75% | 50% |
| Flowlac 90 | 45% | | 25% |
| MagnesiumStearat | 0,5% | 0,5% | 0,5% |

| | | | |
|---|---|---|---|
| * Direktverpressung | | | |

In Tabelle 5 ist die Fließeigenschaft der Formulierungen mit dem Wirkstoff Theophyllin zusammengefasst.

Es erfüllen nur die beiden Formulierungen mit dem erfindungsgemäßen Granulat W2 und W3 die Anforderung an direktverpressbare Formulierungen hinsichtlich der Fließeigenschaften.

**Tabelle 5: Fließeigenschaft der Formulierung mit Wirkstoff Theophyllin**

| Formulierung | Auslaufmenge sec/100g bei Trichteröffnung | |
|---|---|---|
| | d=10 mm | d=15 mm |
| W1 | -* | -* |
| W2 | 29 | 9 |
| W3 | 24 | 8 |

| | | |
|---|---|---|
| -* Formulierung fließt nicht durch Trichter | | |

### 3.3 Tabletten mit Wirkstoff Metformin HCl

Die in Proben M1 und M3 (Tabelle 6) aufgeführten Komponenten (außer Magnesiumstearat) werden im jeweiligen Gewichtsverhältnis in einem Turbula Mischer 5 Minuten gemischt. Magnesiumstearat wird zugegeben und wiederum eine Minute gemischt.

Die erhaltene Mischung wird anschließend bei dem in Tabelle 6 angegebenen Verpressungsdruck direkt verpresst.

Zum Vergleich wird in Probe M2 zunächst die physikalische Mischung bestehend aus HPMC (Benecel), Granulac 200 (Standardware für Nassgranulation) und Wirkstoff einer Nassgranulation unterworfen, bevor das erhaltene Granulat mit Magnesiumstearat vermischt und zu Tabletten verpresst wird. Die jeweiligen Tablettenhärten sind in Tabelle 6 angegeben.

**Tabelle 6**

| Probe | M1 | | M2 | | M3 | |
|---|---|---|---|---|---|---|
| Substanz | % | mg | % | mg | % | mg |
| Metformin HCL | 50.0 | 500 | 50.0 | 500.0 | 50.0 | 500 |
| HPMC-Compound Granulat B1 | 49.5 | 495 | | | 43.5 | 435 |
| HPMC (Benecel) | | | 24.75 | 247.5 | | |
| Granulac 200 | | | 24.75 | 24.75 | | |
| Klucel EXF | | | | | 5.0 | 50 |
| Aerosil | | | | | 1.0 | 10 |
| Magnesium Stearat | 0.5 | 5 | 0.5 | 5 | 0.5 | 5 |
| Gesamt | 100 | 1000 | 100 | 1000 | 100 | 1000 |
| Herstellung | Direktverpressung | | Nassgranulation | | Direktverpressung | |
| Verpressungsdruck (KN) | 27 | | 29 | | 28 | |
| Tablettenhärte (N) | 45 | | 54 | | 87 | |

Wie sich aus Tabelle 6 ergibt, zeigen die direkt verpressten Tabletten M1 in etwa gleiche Tablettenhärte wie die Tabletten M2 der physikalischen Mischung, die über den Zwischenschritt der Nassgranulation hergestellt werden müssen. Eine Direkttablettierung der physikalischen Mischung M2 ist nicht möglich.

Durch teilweise Substitution des Granulats B1 durch die weiteren Hilfsmittel Klucel EXF (Hydroxypropylcellulose) und Aerosil kann ferner eine deutliche Steigerung der Tablettenhärte (und damit der Abriebfestigkeit) gegenüber M1 oder M2 erreicht werden. Formulierung M3 lässt sich ohne Probleme direkt verpressen.

### 3.4 Tablettierung

Die Tablettierung erfolgt auf einer Korsch EK 0, Germany (Tablettenstempel: oblong 22x11 mm Tablettengewicht 1000 mg)

### 3.5 Ergebnisse

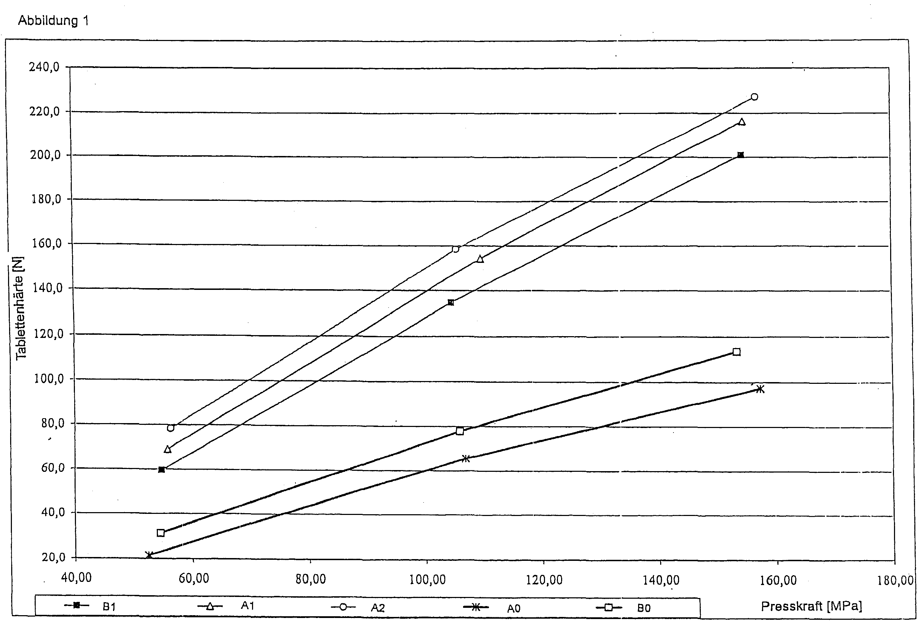

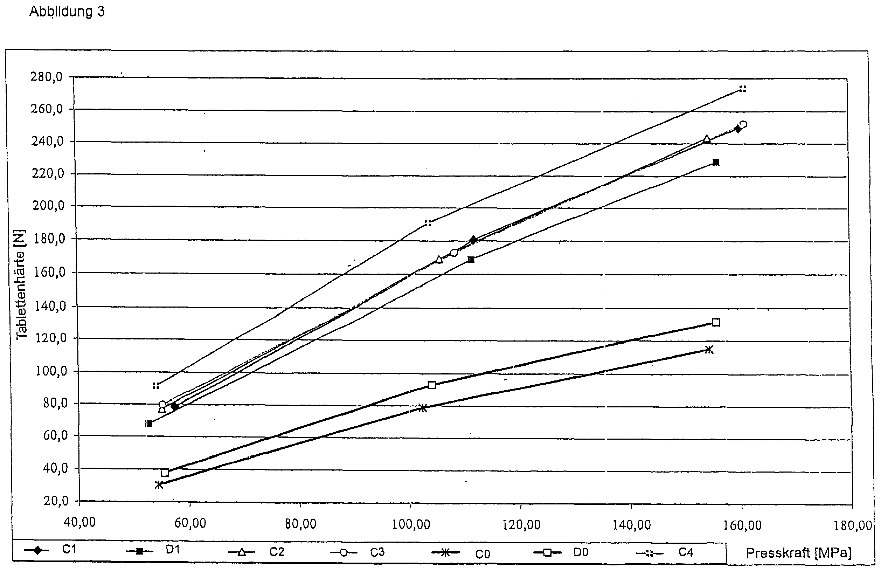

In Abb. 1 und 3 ist die Tablettenhärte der Beispiele A bis D in Abhängigkeit von der Presskraft aufgetragen. Alle Beispiele, bei denen das erfindungsgemäße Granulat als Direkttablettierhilfsstoff bei der Tablettierung eingesetzt wurde, haben eine größere Tablettenhärte im Vergleich zu Tabletten die unter gleichen Bedingungen, aber mit der physikalischen Mischung der Granulatbestandteile, hergestellt wurden.

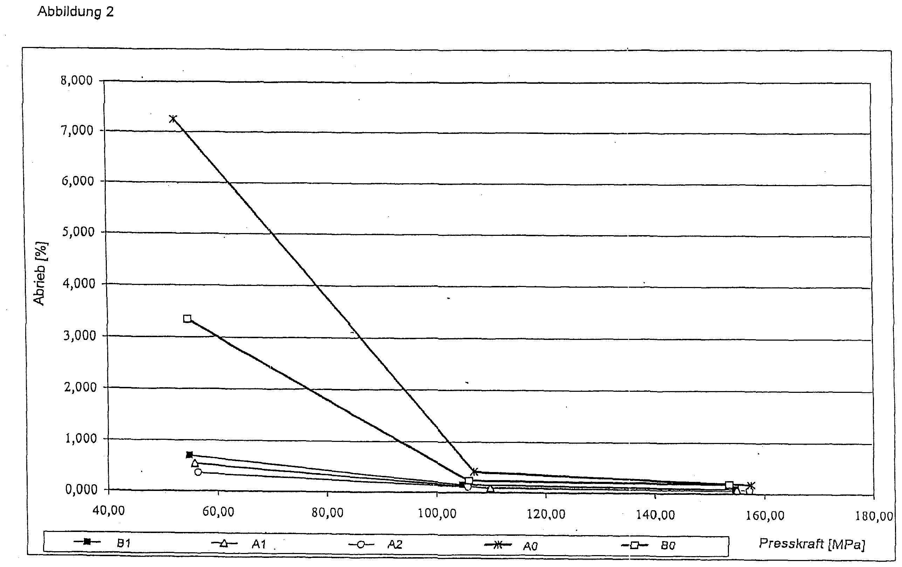

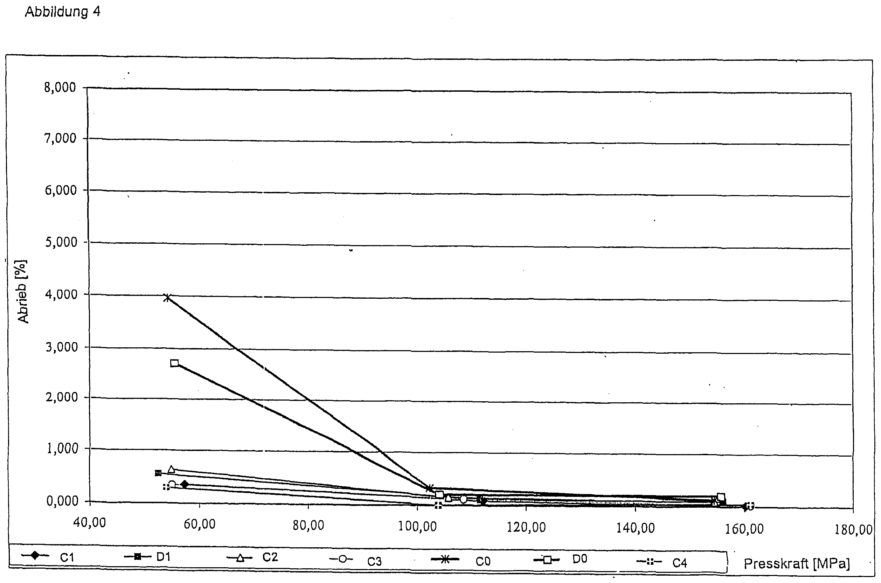

In Abb. 2 und 4 ist die Abriebfestigkeit der Tabletten A bis D in Abhängigkeit von der Presskraft aufgetragen. Alle Beispiele, bei denen das erfindungsgemäße Granulat als Direkttablettierhilfsstoff bei der Tablettierung eingesetzt wurde, zeigen weniger Abrieb im Vergleich zu Tabletten die unter gleichen Bedingungen, aber mit der physikalischen Mischung der Granulatbestandteile, hergestellt wurden.

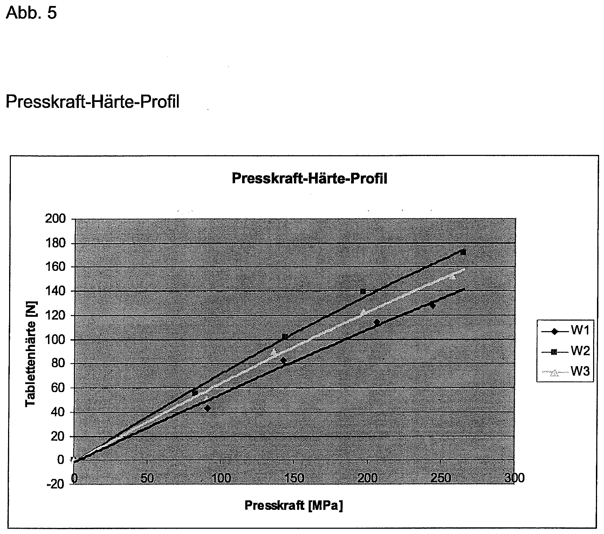

In Abb. 5 ist die Tablettenhärte der Beispiele W1 bis W3 in Abhängigkeit von der Presskraft aufgetragen. Die größte Härteausbeute wird mit Granulat E in der Wirkstoffformulierung erzielt. Durch Zugabe von sprühgetrockneter Laktose (W3) kann die Härte modifiziert werden.

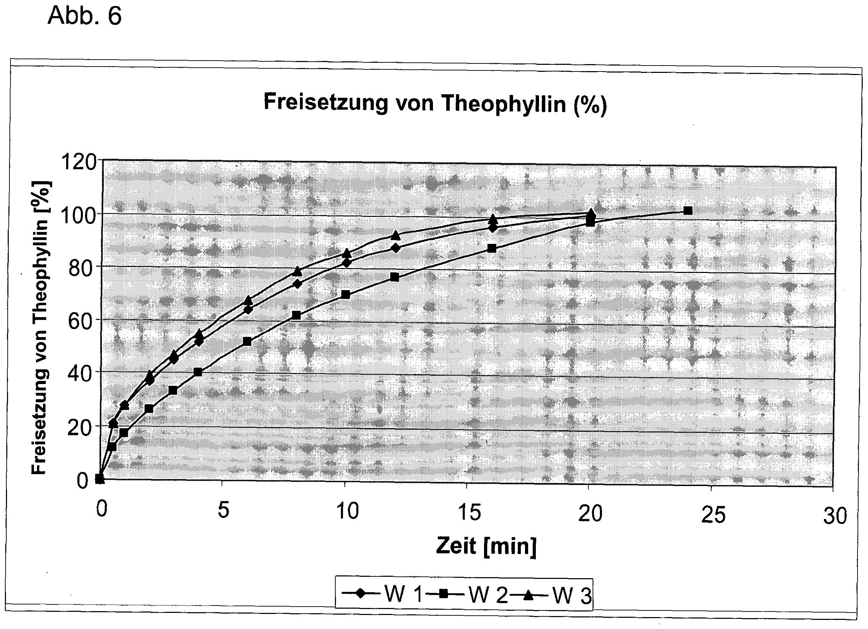

In Abb. 6 ist die Freisetzung von Theophyllin aus den Tabletten W1 bis W3 über die Zeit dargestellt. Die Tabletten wurden hierzu in 0,05 molare Phosphatpufferlösung mit einem pH-Wert von 6,8 gegeben. Aus Abb. 6 kann entnommen werden, dass das Granulat zu einer verzögerten Freisetzung des Wirkstoffs gegenüber der physikalischen Mischung führt. Durch Zugabe von weiteren Hilfsstoffen, wie z.B. von sprühgetrockneter Laktose (W3), kann das Freisetzungsprofil modifiziert werden.

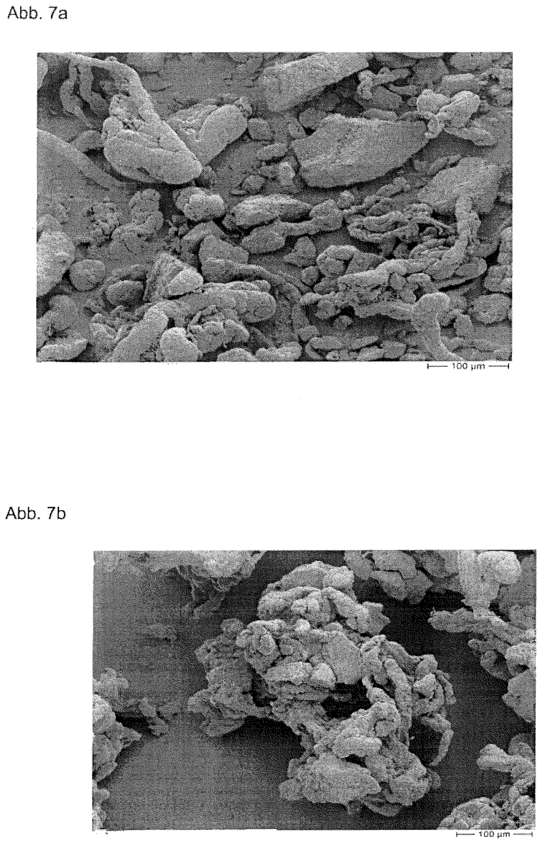

Abbildung 7 zeigt REM-Aufnahmen der physikalischen Mischung B0 (Abb. 7a) im Vergleich zum erfindungsgemäßen Granulat B1 (Abb. 7b). Aus den Bildern geht hervor, dass durch das erfindungsgemäße Verfahren die feinteiligen Ausgangsmaterialien gemäß der physikalischen Mischung zu größeren sphäroiden Granulatkörnern geformt werden.

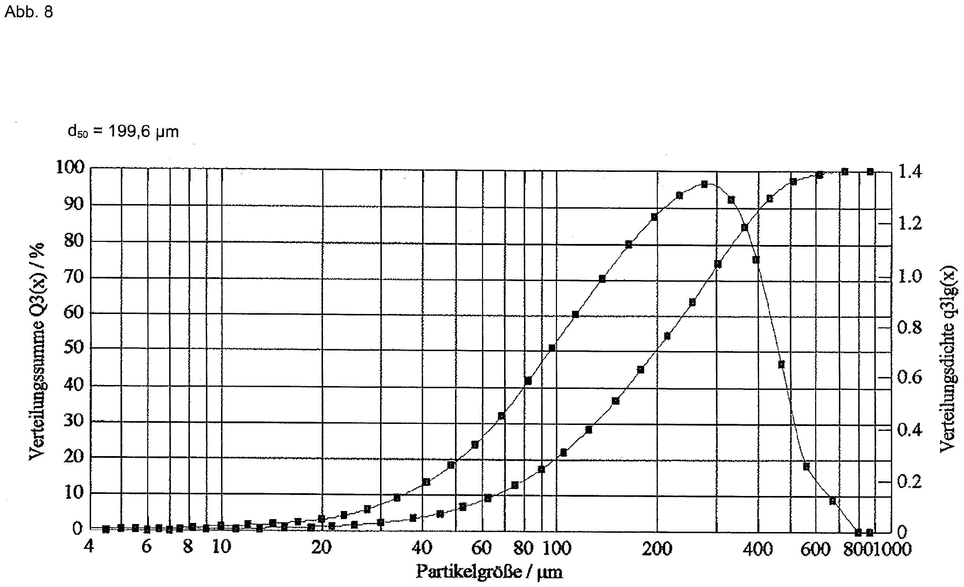

Die Partikelgrößenverteilung in Granulat B1 ist in Abb. 8 dargestellt. Hieraus ergibt sich ein d₅₀-Wert von etwa 200 µm.

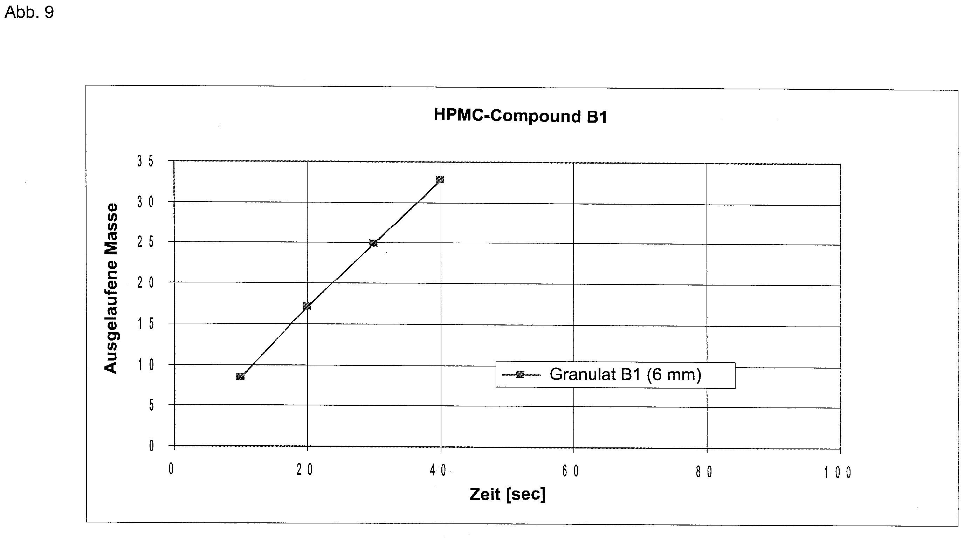

Abbildung 9 gibt das Fließverhalten des Granulats B1 wieder. Dabei ist die aus dem Trichter ausgelaufene Granulatmenge über die Zeit aufgetragen. Die entsprechende physikalische Zusammensetzung B0 kann nicht vermessen werden, da die Formulierung den Trichter vollends verstopft.

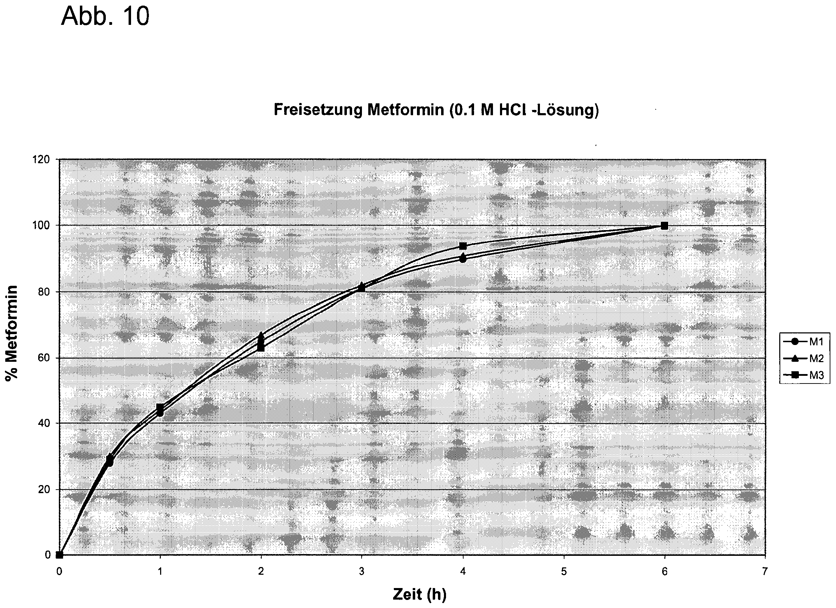

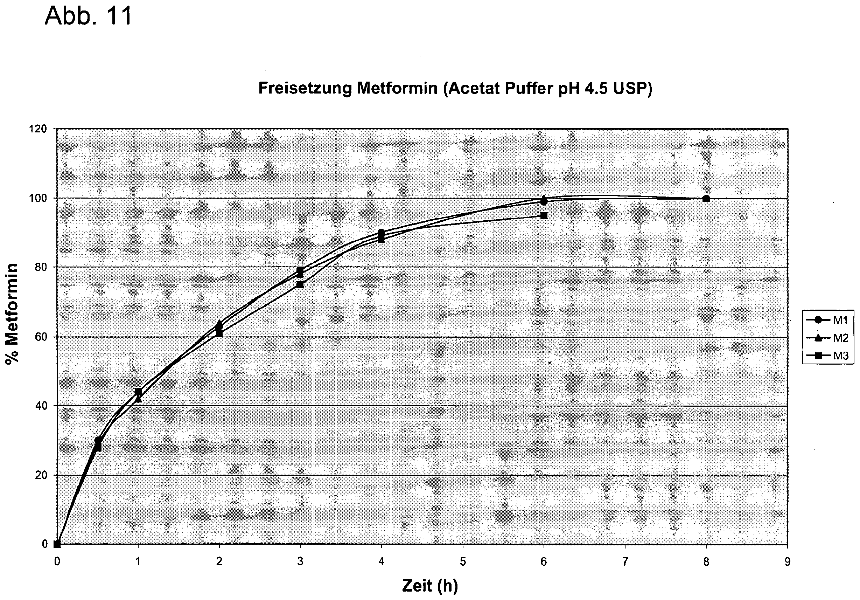

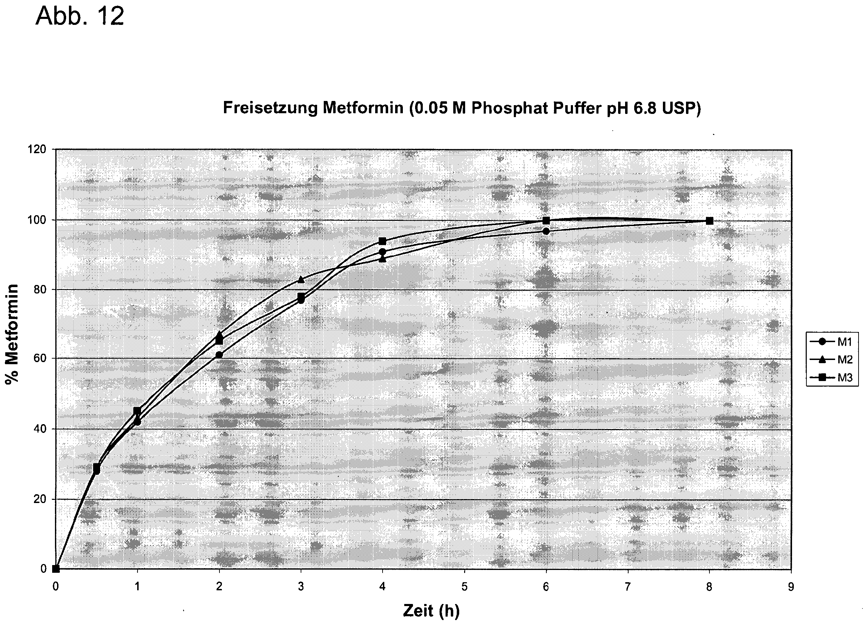

In den Abbildungen 10 bis 12 sind die Ergebnisse der Freisetzungsversuche von Metformin aus den Tabletten M1 bis M3 graphisch dargestellt. Die Freisetzungsversuche erfolgten jeweils in 0.1 M HCl sowie in einem Acetat- bzw. Phosphatpuffer. Wie aus den Graphen hervorgeht, zeigen die Tabletten M1 bis M3 ein vergleichbares Freisetzungsprofil. Unterschiede im Freisetzungsprofil zwischen der Direktverpressung (M1, M3) und der über das Nassgranulationsverfahren hergestellten Probe M2 können nicht beobachtet werden.

## Patentansprüche

1. Verfahren zur Herstellung eines Granulats umfassend die Schritte
i) Suspendieren oder/und zumindest teilweises Lösen von Laktose in einer Flüssigkeit, ausgewählt aus Wasser oder einem organischen Lösungsmittel, und
ii) Zerstäuben der in i) erhaltenen Lösung oder Suspension in eine Umgebung mit einer Temperatur von 30-250 °C auf Cellulosederivat-Partikel und gegebenenfalls Laktose-Partikel, wobei die Flüssigkeit zumindest teilweise entfernt wird.

2. Verfahren nach Anspruch 1, **dadurch gekennzeichnet, dass** die Laktose ausgewählt ist aus der Gruppe bestehend aus Laktose Monohydrat und wasserfreier Laktose, insbesondere Laktose Monohydrat.

3. Verfahren nach einem der Ansprüche 1 bis 2, **dadurch gekennzeichnet, dass** das Cellulosederivat ausgewählt ist aus der Gruppe bestehend aus Cellulose, deren Hydroxylgruppen unabhängig voneinander zumindest teilweise alkyliert, hydroxyalkyliert, sulfoniert, carboxyalkyliert oder/und xanthogeniert sind, inbesondere Hypromellose (HPMC), Hypromellosephthalat, Hydroxypropylcellulose (HPC), Hydroxyethylcellulose, Ethylcellulose (EC), Carboxymethylcellulose (CMC), Carboxyethylcellulose (CEC) oder/und deren Natrium- oder/und Calciumsalze.

4. Verfahren nach einem der Ansprüche 1 bis 3, **dadurch gekennzeichnet, dass** die Lösung oder Suspension im Schritt ii) über eine Düse zu Tröpfchen mit einem durchschnittlichen Durchmesser von 15 bis 1250 µm zerstäubt wird.

5. Verfahren nach einem der Ansprüche 1 bis 4, **dadurch gekennzeichnet, dass** die Lösung oder Suspension in eine Umgebung mit einer Temperatur von 40 - 170°C zerstäubt wird, in der insbesondere ein Druck von 0 - 1,0 bar, bevorzugt von 0,003 - 0,7 bar, herrscht.

6. Verfahren nach einem der Ansprüche 1 bis 5, **dadurch gekennzeichnet, dass** die Cellulosederivat- oder/und Laktose-Partikel einen durchschnittlichen Durchmesser von 1 µm bis 500 µm haben.

7. Verfahren nach einem der Ansprüche 1 bis 6, **dadurch gekennzeichnet, dass** das Verhältnis der Cellulosederivat- zu Laktose-Partikel im Schritt ii) im Bereich von 100/0 bis 5/95 liegt.

8. Verfahren nach einem der Ansprüche 1 bis 7, **dadurch gekennzeichnet, dass** die Flüssigkeit über ein Sprühtrocknungsverfahren, ein Wirbelschichtgranulationsverfahren oder ein Feuchtgranulationsverfahren zumindest teilweise entfernt wird.

9. Verfahren nach einem der Ansprüche 1 bis 8, **dadurch gekennzeichnet, dass** die Gesamtheit der Cellulosederivat- und gegebenenfalls Laktose-Partikel in einem Fließbett oder einer Wirbelschicht vorliegen.

10. Verfahren nach einem der Ansprüche 1 bis 9, **dadurch gekennzeichnet, dass** das erhaltene Granulat ein Verhältnis von Laktose / Cellulosederivat zwischen 95/5 bis 1/99, bevorzugt 90/10 bis 5/95 aufweist, wobei der Gehalt an freier Flüssigkeit im Granulat insbesondere bei < 8 Gew.-% bezogen auf die Gesamtmasse des Granulats liegt.

11. Verfahren nach einem der Ansprüche 1 bis 10, dadurchgekennzeichnet, dass das Granulat eine sphärische oder sphäroide Morphologie aufweist, insbesondere mit einer d₅₀-Partikelgrößenvertellung von 25 - 750 µm.

12. Granulat erhältlich durch ein Verfahren nach einem der Ansprüche 1 bis 11.

13. Zusammensetzung umfassend Granulat nach Anspruch 12, mindestens eine pharmazeutisch aktive Komponente und gegebenenfalls weitere Hilfsstoffe, insbesondere Schmiermittel, Füllmittel, Bindemittel, Gleitmittel, Fließregulierungsmittel, Antistatika, Lösungsvermittler und Feuchthaltemittel.

14. Verwendung des Granulats nach Anspruch 12 als Tablettierhilfsstoff oder Direkttablettierhilfsstoff.

## Claims

1. Method for producing a granulate, comprising the steps of:
i) suspending and/or at least partially dissolving lactose in a liquid selected from water or an organic solvent, and
ii) atomising the solution or suspension obtained in i) in an environment at a temperature of from 30 - 250 °C onto cellulose-derivative particles and optionally lactose particles, the liquid being at least partially removed.

2. Method according to claim 1, **characterised in that** the lactose is selected from the group consisting of lactose monohydrate and anhydrous lactose, in particular lactose monohydrate.

3. Method according either claim 1 or claim 2, **characterised in that** the cellulose derivative is selected from the group consisting of cellulose, the hydroxyl groups of which are, independently of one another, at least partially alkylated, hydroxyalkylated, sulfonated, carboxyalkylated and/or xanthogenated, in particular hypromellose (HPMC), hypromellose phthalate, hydroxypropyl cellulose (HPC), hydroxyethyl cellulose, ethyl cellulose (EC), carboxymethyl cellulose (CMC), carboxyethyl cellulose (CEC) and/or the sodium and/or calcium salts thereof.

4. Method according to any of claims 1 to 3, **characterised in that** the solution or suspension is atomised in step ii) by a nozzle to form droplets having an average diameter of from 15 to 1250 µm.

5. Method according to any of claims 1 to 4, **characterised in that** the solution or suspension is atomised in an environment at a temperature of from 40 - 170 °C in which a pressure in particular of from 0 - 1.0 bar, preferably of from 0.003 - 0.7 bar, prevails.

6. Method according to any of claims 1 to 5, **characterised in that** the cellulose derivative particles and/or lactose particles have an average diameter of from 1 µm to 500 µm.

7. Method according to any of claims 1 to 6, **characterised in that** the ratio of cellulose derivative particles to lactose particles in step ii) is in a range of from 100/0 to 5/95.

8. Method according to any of claims 1 to 7, **characterised in that** the liquid is at least partially removed by means of a spray-drying process, a fluidised-bed granulation process or a wet-granulation process.

9. Method according to any of claims 1 to 8, **characterised in that** all of the cellulose derivative particles and optionally lactose particles are present in a fluid bed or a fluidised bed.

10. Method according to any of claims 1 to 9, **characterised in that** the obtained granulate has a ratio of lactose/cellulose derivative of from 95/5 to 1/99, preferably from 90/10 to 5/95, the free liquid content in the granulate being in particular < 8 wt.%, based on total mass of the granulate.

11. Method according to any of claims 1 to 10, **characterised in that** the granulate has a spherical or spheroid morphology, in particular having a d₅₀ particle size distribution of from 25 - 750 µm.

12. Granulate obtained by a method according to any of claims 1 to 11.

13. Composition comprising granulate according to claim 12, at least one pharmaceutically active component, and optionally further excipients, in particular lubricants, fillers, binders, slip additives, flow regulation agents, antistatic agents, solubilisers and humectants.

14. Use of the granulate according to claim 12 as a tabletting excipient or direct tabletting excipient.

## Revendications

1. Procédé de fabrication de granulés, comprenant les étapes suivantes :
i) suspension et/ou au moins partiellement dissolution de lactose dans un liquide sélectionné parmi l'eau ou un solvant organique, et
ii) pulvérisation de la solution ou de la suspension obtenue dans i) dans un environnement avec une température de 30-250 °C sur des particules de dérivé de cellulose et éventuellement de particules de lactose, le liquide étant enlevé au moins partiellement.

2. Procédé selon la revendication 1, **caractérisé en ce que** le lactose est sélectionné parmi le groupe composé de lactose monohydraté et de lactose exempt d'eau, en particulier de lactose monohydraté.

3. Procédé selon l'une des revendications 1 à 2, **caractérisé en ce que** le dérivé de cellulose est sélectionné dans le groupe composé de cellulose, dont les groupes hydroxyle sont au moins partiellement alkylés, hydroalkylés, sulfonés, carboxyalkylés ou/et xanthogénés indépendamment les uns des autres, en particulier hypromellose (HPMC), hypromellosepthalate, hydroxypropylcellulose (HPC), hydroxyéthylcellulose, éthylcellulose (EC), carboxyméthylcellulose (CMC), carboxyéthylcellulose (CEC) ou/et leurs sels de sodium ou/et de calcium.

4. Procédé selon l'une des revendications 1 à 3, **caractérisé en ce que** la solution ou la suspension dans l'étape ii) est, par le biais d'une buse, pulvérisée en gouttelettes d'un diamètre moyen de 15 à 1 250 µm.

5. Procédé selon l'une des revendications 1 à 4, **caractérisé en ce que** la solution ou la suspension est pulvérisée dans un environnement à une température de 40 - 170 °C dans lequel règne en particulier une pression de 0 - 1,0 bar, de préférence de 0,003 à 0,7 bar.

6. Procédé selon l'une des revendications 1 à 5, **caractérisé en ce que** les particules de dérivé de cellulose ou/et de lactose ont un diamètre moyen de 1 µm à 500 µm.

7. Procédé selon l'une des revendications 1 à 6, **caractérisé en ce que** le rapport des particules de dérivé de cellulose aux particules de lactose dans l'étape ii) se situe dans la plage de 100/0 à 5/95.

8. Procédé selon l'une des revendications 1 à 7, **caractérisé en ce que** le liquide est enlevé au moins partiellement par le biais d'un procédé de séchage par pulvérisation, un procédé de granulation en couche fluidisé ou un procédé de granulation humide.

9. Procédé selon l'une des revendications 1 à 8, **caractérisé en ce que** la totalité des particules de dérivé de cellulose et éventuellement des particules de lactose sont présentes dans un lit fluidisé ou une couche fluidisée.

10. Procédé selon l'une des revendications 1 à 9, **caractérisé en ce que** les granulés obtenus présentent un rapport de lactose / dérivé de cellulose entre 95/5 et 1/99, de préférence entre 90/10 et 5/95, la teneur en liquide libre dans les granulés se situant en particulier à < 8 %-masse, rapportée à la masse totale des granulés.

11. Procédé selon l'une des revendications 1 à 10, **caractérisé en ce que** les granulés présentent une morphologie sphérique ou sphéroïde, en particulier avec une distribution de tailles de particules d₅₀ de 25 - 750 µm.

12. Granulés pouvant être obtenus par un procédé selon l'une des revendications 1 à 11.

13. Composition comprenant des granulés selon la revendication 12, au moins un composé pharmaceutiquement actif et éventuellement d'autres additifs, en particulier des lubrifiants, des charges, des liants, des agents de glissement, des agents de régulation de l'écoulement, des agents antistatiques, des agents de solubilisation et des agents humectants.

14. Utilisation des granulés selon la revendication 12 en tant qu'additif de pastillage ou en tant qu'auxiliaire de pastillage direct.
